(19) Europäisches Patentamt
European Patent Office
Office européen des brevets

(11) EP 2 422 815 A1

(12) EUROPEAN PATENT APPLICATION

(43) Date of publication:
29.02.2012 Bulletin 2012/09

(51) Int Cl.:
A61K 45/06 (2006.01)  A61K 31/513 (2006.01)
A61K 31/353 (2006.01)  A61P 35/00 (2006.01)

(21) Application number: 10173257.6

(22) Date of filing: 18.08.2010

(84) Designated Contracting States:
AL AT BE BG CH CY CZ DE DK EE ES FI FR GB
GR HR HU IE IS IT LI LT LU LV MC MK MT NL NO
PL PT RO SE SI SK SM TR
Designated Extension States:
BA ME RS

(71) Applicant: Grindeks, a joint stock company
1057 Riga (LV)

(72) Inventors:
• Kalvins, Ivars
1006 Riga (LV)

• Stonans, Ilmars
1057 Riga (LV)
• Sestakova, Irina
1006 Riga (LV)
• Domracova, Ilona
1006 Riga (LV)
• Jascenko, Elina
1006 Riga (LV)
• Bridane, Veronika
1006 Riga (LV)
• Kanepe, Iveta
1006 Riga (LV)

(54) **Pharmaceutical composition of tegafur and natural flavonoid derivative catechin for potentiating antitumour effect and for treating tumours**

(57) Novel pharmaceutical composition for treating cancer, preferably malignant cancer by way of enteral use have been described, which contain natural flavonoid derivative Catechin and cancer chemotherapeutic agent TEGAFUR

EP 2 422 815 A1

**Description**

Technical Field

The present invention relates to a novel composition of TEGAFUR and natural flavonoid derivative Catechin (Ca) for potentiating anti-tumour effect and for treating tumours; in particular, it relates to an unexpected synergism of a combination of natural flavonoid derivative and an anti-tumour agent TEGAFUR in the treatment of cancer; and to processes for preparing the composition and its use for chemotherapy of tumours, especially malignant tumours.

Background Art

More than 11 million people are diagnosed with cancer every year. It is estimated that there will be 16 million new cases every year by 2020. Cancer causes 7 million deaths every year - or 12.5% of deaths worldwide.

Therefore, a great number of investigations, developments and research works have been carried out to find anticancer agents, which can be used in chemotherapy of malignant tumours. There are known various anticancer agents and results of cancer treatment improve with every year, nevertheless the amount of effective dose of anticancer agent for treatment is still high.

One of such anticancer agents is TEGAFUR. TEGAFUR, i.e. 1-(2-tetrahydrofuryl)-5-fluorouracil, is disclosed is GB 1 168 391 (INST ORGANOCHESKOGO SINTEZA, published 22.10.1969) as a prodrug of 5-FU, i.e. 5-fluorouracil, and proved active in the management of metastatic colorectal cancer. TEGAFUR is metabolized to 5-fluorouracil (5-FU) in vivo, predominantly in the liver, and has been reported to be less toxic and to have a higher therapeutic index CAO than 5-fluorouracil prodrug. It plays a role in anabolic and catabolic pathway modulation in therapy of colorectal cancer (see "Cancer Research" 1995, vol. 1, p.839-845).

US 4 328 229 (TAIHO PHARMACEUTICAL, published 04.05.1982) discloses an anti-cancer composition, containing 1-(2-tetrahydrofuryl)-5-fluorouracil (TEGAFUR) and uracil. The composition is used for delivery of 5-fluorouracil to a tumour which is sensitive to 5-fluorouracil in a warm-blooded animal. It is disclosed therein that the composition can be co-administered in a variety of dosage forms including an oral dosage form.

Flavonoids are polyphenolic secondary metabolites widely dispersed throughout the plant kingdom and found in substantial levels in commonly consumed fruits, vegetables and beverages. The flavonoids, which are benzo-{gamma}-pyrone derivates with A, B and C rings, are categorized as: flavonols, flavones, flavan-3-ols, isoflavones, flavanones and anthocyanidins. They have been shown to possess a variety of biological activities at non-toxic concentrations in organisms. The role of dietary flavonoids in cancer prevention is widely discussed. Compelling data from laboratory studies, epidemiological investigations, and human clinical trials indicate that flavonoids have important effect on cancer chemoprevention and chemotherapy. Many mechanisms of action have been identified, including carcinogen inactivation, antiproliferation, cell cycle arrest, induction of apoptosis and differentiation, inhibition of angiogenesis, antioxidation and reversal of multidrug resistance or a combination of these mechanisms. Based on these results, flavonoids may be promising anticancer agents, published in "Medical Research Review" 2003, vol.23, no. 4, p. 519-534.

Catechins are polyphenolic antioxidant plant metabolites, they are specially flavonoids called flavan-3-ols.

Catechins have potent antioxidant and cancer chemopreventive properties. These compounds are found in a variety of plants and are present in particularly high amounts in tea leaves, published in "Cancer research" 2001, no. 61, p.118-125.

Green tea and, to a lesser extent, black tea are rich sources of catechins. Studies by Japanese researchers have shown that catechins can prevent free radical damage of cholesterol and lower the risk of heart disease and cancer, published in http://www.thenutritionreporter.com/power_of_flavonoids.html 03/07/2007

High levels of monomeric (+)-catechin are found in the skin and seeds of fruits such as apples and grapes. Red wine and chocolate also are significant sources of (+)-catechin. In keeping with the ability of phenolic antioxidants to induce the expression of phase II detoxifying enzymes in mammalian cells, (+)-catechins produces antimutagenic effects, published in "Cancer research" 2001, no. 61, p.118-125.

KR20020045417 (published 19.06.2002) describes polypehnol compound, that is extracted from green tea. It contains catechins, such as (+)-catechin and it can be effectively used as an anticancer agent for treatment and prevention of a tumour because it induces the growth inhibition and death of human cancer cells by apoptosis.

Disclosure of Invention

Technical problem

In malignant tumour treatment different kind of pharmaceutical compositions are used, including pharmaceutical compositions of TEGAFUR, but just only partial remission of malignant tumour was obtained.

As well as composition which is disclosed in WO 2005105086 (TAIHO PHARMACEUTICAL CO LTD) 11.10.2005 said that it reducing the gastrointestinal toxicity of TEGAFUR and improving tolerability of 5-Fu, but still only partial remission of tumour is developed.

The further aim of present invention is to developed effective pharmaceutical composition that can be used in malignant tumour treatment for achieving increased remission of tumours.

Technical solution

The present invention is directed to a composition for treating a tumour, especially a malignant tumour in an individual who need such treatment, comprising administration to said individual of a pharmacologically effective dose of one of natural flavonoid derivative, preferably Catechin and the cancer chemotherapeutic agent TEGAFUR.

While attempting to develop a pharmaceutical composition for malignant tumour treatment having an essentially lower toxicity, we unexpectedly found that doses adequate to the therapeutic ones used in the clinical use of pharmaceutical compositions containing TEGAFUR [1-(2-tetrahydrofuryl)-5-fluorouracil], a drug for treating a malignant tumour, the co-administration or combined use in pharmaceutical compositions of a compound of the natural flavnonoid derivative, preferably Catechin.

Therefore, the synergistic effect of natural flavonoid derivative Catechin in combination with TEGAFUR is unexpected and surprising.

By using a composition of Catechin and TEGAFUR the following advantageous effect are attained:

■ Tegafur potentiating anti-tumour effect of natural flavonoid derivate in the treatment of tumours;

■ The use of a combination of Tegafur and one of natural flavonoid derivative, preferably (+)-Catechin, allows efficacy increase growth inhibition of S-180 ascites tumour.

There is no particular restriction on the unit dosage from which can be adopted for the anti-tumour composition of the invention in the treatment of malignant tumours in mammals. The unit dosage form is selected according to the purpose of treatment. Examples thereof are oral dosage form such as tablets, coated tablets, pills, powders, granules, capsules, solutions, suspensions, emulsions, etc. and parenteral dosage forms such as suppositories, ointments, plasters, etc. These dosage dorms can be manufactured by convention pharmaceutical procedures known in this field. As the carrier for shaping into the form of tablets, there can be employed various excipients such as lactose, sucrose, sodium, glucose, urea, starch, calcium carbonate, kaolin, crystalline cellulose, silicic acid, etc.; binders such as simple syrup, glucose solution, starch solution, gelatine solution, carboxymethylcellulose, shellac, methylcellulose, potassium phosphate, polyvinylpyrrolidone, etc,; disintegrators such as dry starch, sodium alginate, agar powder, laminaran powder, sodium hydrogen carbonate, calcium carbonate, polyoxyethylene-sorbitan fatty acid esters, sodium lauryl sulphate, stearic acidmonoglyceride, starch, lactose, etc.; antidisintegrators such as sucrose, stearic acid, cacao butter, hydrogenated oil etc.; absorption promotors such as quaternary ammonium bases, sodium lauryl sulphate, etc.; humectants such as glycerol, starch, etc.; adsorbents such as starch, lactose, kaolin, bentonite, colloidal silicic acid, etc.; and lubricants such as purified talc, stearic acid salts. Boric acid powder, polyethylene glycol, etc. Where necessary, the tablets may be in the form of coated tablets such as sugar-coated tablets, gelat-coated tablets, enteric-coated tablets, film-coated tablets, or double or multi-layer tablets, etc.

The carrier for shaping into the form of pills includes, for example, various excipients such as glucose, lactose, starch, cacao butter, hardened vegetable oil, kaolin, talc, etc.; and disintegrators such as laminarian, agar, etc. The carrier for shaping into the form of suppositories includes, for example, polyethylene glycol, cacao butter, higher alcohols, esters of higher alcohols, gelatine, semi-synthetic glycerides, etc.

Capsules are manufactured by mixing the natural flavonoid derivative, preferably Catechin with TEGAFUR, with any of the carriers mentioned above and encapsulating the mixture in hard gelatine capsule, soft capsule or other capsules.

For manufacturing in the form of pastes, creams and gels, there is employed a diluent such as, for example, white petrolatum, paraffin, glycerol, cellulose derivatives, polyethylene glycols, silicone, bentonite, etc.

Best Mode for Carrying Out the Invention

The present invention in the following will be described in more detail with reference to pharmacological tests and examples illustrating the preparation of the anti-tumour effect-potentiating compositions of the invention comprising natural flavonoid derivative Catechin and TEGAFUR.

PHARMACOLOGICAL TEST 1

Experimental example

Experiments in vivo were carried out on mice sarcoma S-180 ascites form.

The anti-tumour activity of the pharmaceutical composition of TEGAFUR and one of natural flavonoid derivatives was determined by introducing them into ICR mice of initial weight 20-23 g during a fortnight. Throughout the experiment, the mice were kept under standard laboratory conditions at a temperature of $22\pm2$ °C, relative humidity 55 $\pm$15%, ventilation - 15-20 changes of air amount/hour and a 12 hours light/darkness cycle, and were fed with standard laboratory animal food.

Mice sarcoma S-180 transplanted intraperitoneally to ICR mice in amount 5 x $10^6$ cell/mouse.

Then, 24 hours later, orally administration of compounds was started once a day at the 1st, 3rd, 5th, 7th, 9th, 11th and 13th days.

The compound efficacy was expressed as the percentage of growth inhibition of S-180 ascites tumour, calculated using the equation:

$$\text{Growth inhibition of S-180 ascites tumour}(\%) = 100 - \left(\frac{T}{C}\times100\right)$$

wherein C - mean weight (g) of the control group and T - mean weight (g) of the experimental group.

The results of Table 1 show the growth inhibition of S-180 ascites tumour at 13th day after the transplantation of tumour in mice.

Table 1

| Nr. | Drug | Dosage (mg/kg) | Growth inhibition of S-180 ascites tumour, % |
|---|---|---|---|
| 1 | TEGAFUR | 25 | 9 |
| 2 | Catechin | 100 | 9 |
| 3 | TEGAFUR+ Catechin | 25+100 | 28 |

As it is shown in Table 1 combinations of Tegafur and one of natural flavonoid derivative showed remarkable synergism between the components and allows efficacy increase growth inhibition of S-180 ascites tumour.

Formulation Example 1: Granules

A possible pharmaceutical composition for oral use exemplifying but not exhausting the present invention is the following formulation of granules manufacture:

| | Example1 | Example2 | Example3 | Example4 | Example5 |
|---|---|---|---|---|---|
| Tegafur | 200 mg | 200 mg | 200 mg | 50 mg | 200 mg |
| (+)-Catechin | 50 mg | 50 mg | 200 mg | 100 mg | 10 mg |
| Lactose | 400 mg | 400 mg | 400 mg | 400 mg | 400 mg |
| Corn starch | 400 mg | 400 mg | 400 mg | 400 mg | 400 mg |
| Hydroxymethylcellulose | 10 mg | 10 mg | 10 mg | 10 mg | 10 mg |
| Total | 1110 mg | 1160 mg | 1220 mg | 1160 mg | 1220 mg |

Formulation Example 2: Tablets

A possible pharmaceutical composition for oral use exemplifying but not exhausting the present invention is the following formulation of tablets production:

| | Example1 | Example2 | Example3 | Example4 | Example5 |
|---|---|---|---|---|---|
| Tegafur | 20 mg | 35 mg | 40 mg | 10 mg | 35 mg |
| (+)-Catechin | 20 mg | 15 mg | 5 mg | 5 mg | 10 mg |

(continued)

|  | Example1 | Example2 | Example3 | Example4 | Example5 |
|---|---|---|---|---|---|
| Lactose | 100 mg | 100 mg | 100 mg | 100 mg | 100 mg |
| Crystalline cellulose | 50 mg | 50 mg | 50 mg | 50 mg | 50 mg |
| Magnesium stearate | 10 mg | 10 mg | 10 mg | 10 mg | 10 mg |
| Talc | 10 mg | 10 mg | 10 mg | 10 mg | 10 mg |
| Total | 220 mg | 225 mg | 225 mg | 225 mg | 218.5 mg |

Using the conventional procedure, the tablets were prepared according to the above formula.

Formulation Example 3: Capsules

A possible pharmaceutical composition for oral use exemplifying but not exhausting the present invention is the following formulation of capsules production:

|  | Example 1 | Example 2 | Example 3 | Example 4 | Example 5 |
|---|---|---|---|---|---|
| Tegafur | 100 mg | 100 mg | 50 mg | 150 mg | 50 mg |
| (+)-Catechin | 25 mg | 25 mg | 100 mg | 50 mg | 50 mg |
| Lactose | 50 mg | 50 mg | 50 mg | 50 mg | 50 mg |
| Talc | 50 mg | 50 mg | 50 mg | 50 mg | 50 mg |
| Ca stearate | 6 mg | 7 mg | 5 mg | 7 mg | 7 mg |
| Aerosil | 3 mg | 3 mg | 5 mg | 3 mg | 3 mg |
| Total | 260 mg | 285 mg | 360 mg | 360 mg | 360 mg |

Using the conventional procedure, the capsules were prepared according to the above formula.

Formulation Example 4: Suppository

A possible pharmaceutical composition for internal use exemplifying but not exhausting the present invention is the following formulation of suppository production:

|  | Example 1 | Example 2 | Example 3 | Example 4 | Example 5 |
|---|---|---|---|---|---|
| Tegafur | 250 mg | 250 mg | 300 mg | 100 mg | 100 mg |
| (+)-Catechin | 100 mg | 100 mg | 100 mg | 150 mg | 100 mg |
| Witepsol W-35 | 800 mg | 750 mg | 750mg | 800 mg | 800 mg |
| Total | 1200 mg | 1200 mg | 1200 mg | 1200 mg | 1200 mg |

Using the conventional procedure, suppositories were prepared according to the above formula.

Formulation Example 5: Suspensions

A possible pharmaceutical composition for topical use exemplifying but not exhausting the present invention is the following formulation of suspension production:

|  | Example 1 | Example 2 | Example 3 | Example 4 | Example 5 |
|---|---|---|---|---|---|
| Tegafur | 250 mg | 100 mg | 80 mg | 100 mg | 200 mg |

(continued)

|  | Example 1 | Example 2 | Example 3 | Example 4 | Example 5 |
|---|---|---|---|---|---|
| (+)-Catechin | 50 mg | 200 mg | 80 mg | 50 mg | 50 mg |
| Glycerin | 100 mg | 100 mg | 100 mg | 50 mg | 50 mg |
| Disstilled water | 150 mg | 200 mg | 100 mg | 100 mg | 100 mg |
| Total | 600 mg | 800 mg | 560 mg | 350 mg | 500 mg |

Using the conventional procedure, suppositories were prepared according to the above formula.

Industrial Application

Pharmaceutical composition for treating tumours, preferably malignant tumour which contain natural flavonoid derivative Catechin in clinically efficacious amount and TEGAFUR might be manufactured in a standard pharmaceutical plant.

## Claims

1. A pharmaceutical composition comprising Tegafur [1-(2-tetrahydrofuryl)-5-fluorouracil] or a pharmaceutically acceptable salt thereof and Catechin.

2. A pharmaceutical composition according to claim 1, wherein the preferable ratio of the active ingredients Tegafur and Catechin is from 1:100 to 100:1.

3. A pharmaceutical composition according to claim 1, wherein the preferable ratio of the active ingredients Tegafur and Catechin is from 1:20 to 20:1.

4. A pharmaceutical composition according to claim 1, wherein the preferable ratio of the active ingredients Tegafur and Catechin is from 4:1 to 1:4.

5. Use of pharmaceutical composition of any preceding claim as a medicament.

6. A pharmaceutical composition according to claims 1-4 for use in the treatment of a tumour.

7. Use of a pharmaceutical composition according to claims 1-4 for the manufacture of a medicament for administration simultaneously, sequentially or separately for treatment and/or prevention of tumour.

Europäisches
Patentamt

European
Patent Office

Office européen
des brevets

# EUROPEAN SEARCH REPORT

Application Number

EP 10 17 3257

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2008/075201 A (UNIV MURCIA [ES]; RODRIGUEZ-LOPEZ JOSE NEPTUNO [ES]; CABEZAS-HERRERA J) 26 June 2008 (2008-06-26) * page 23, lines 22-27 * * page 5, line 28 - page 6, line 15; claims 5,6 * | 1-7 | INV. A61K45/06 A61K31/513 A61K31/353 A61P35/00 |
| A | NAVARRO-PERAN ET AL: "Effects of folate cycle disruption by the green tea polyphenol epigallocatechin-3-gallate" INTERNATIONAL JOURNAL OF BIOCHEMISTRY AND CELL BIOLOGY, EXETER, GB, vol. 39, no. 12, 1 January 2007 (2007-01-01), pages 2215-2225, XP022277795 ISSN: 1357-2725 * page 2219, right-hand column; figure 4 * | 1-7 | |
| A | SUN Z -J ET AL: "Anti-hepatoma activity of resveratrol in vitro" WORLD JOURNAL OF GASTROENTEROLOGY 2002 CN, vol. 8, no. 1, 2002, pages 79-81, XP002505236 ISSN: 1007-9327 * page 80, right-hand column, paragraph 2 * | 1-7 | TECHNICAL FIELDS SEARCHED (IPC) A61K A61P |
| A | TAKANO FUMIHIDE ET AL: "Protective effect of (+)-catechin against 5-fluorouracil-induced myelosuppression in mice" TOXICOLOGY, vol. 201, no. 1-3, 1 September 2004 (2004-09-01), pages 133-142, XP002505237 ISSN: 0300-483X * abstract * | 1-7 | |

-/--

The present search report has been drawn up for all claims

| Place of search | Date of completion of the search | Examiner |
|---|---|---|
| Munich | 9 November 2010 | Herrera, Suzanne |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

................................................................

& : member of the same patent family, corresponding
    document

EPO FORM 1503 03.82 (P04C01)

Europäisches
Patentamt

European
Patent Office

Office européen
des brevets

# EUROPEAN SEARCH REPORT

**Application Number**

EP 10 17 3257

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | GARG A K ET AL: "Chemosensitization and radiosensitization of tumors by plant polyphenols" ANTIOXIDANTS AND REDOX SIGNALING, MARY ANN LIEBERT, LARCHMONT, NY, US, vol. 7, no. 11-12, 1 January 2005 (2005-01-01), pages 1630-1647, XP009108932 ISSN: 1523-0864 * table 1 * | 1-7 | |
| A | HOFF P M: "THE TEGAFUR-BASED DIHYDROPYRIMIDINE DEHYDROGENASE INHIBITORY FLUOROPYRIMIDINES, UFT/LEUCOVORIN (ORZEL) AND S-1: A REVIEW OF THEIR CLINICAL DEVELOPMENT AND THERAPEUTIC POTENTIAL" INVESTIGATIONAL NEW DRUGS, MARTINUS NIJHOFF PUBLISHERS, BOSTON, US, vol. 18, no. 4, 1 January 2000 (2000-01-01), pages 331-342, XP009062208 ISSN: 0167-6997 * page 339, right-hand column * | 1-7 | |

TECHNICAL FIELDS
SEARCHED (IPC)

The present search report has been drawn up for all claims

| Place of search | Date of completion of the search | Examiner |
|---|---|---|
| Munich | 9 November 2010 | Herrera, Suzanne |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 10 17 3257

This annex lists the patent family members relating to the patent documents cited in the above-mentioned European search report.
The members are as contained in the European Patent Office EDP file on
The European Patent Office is in no way liable for these particulars which are merely given for the purpose of information.

09-11-2010

| Patent document cited in search report | Publication date | Patent family member(s) | Publication date |
|---|---|---|---|
| WO 2008075201 A | 26-06-2008 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- GB 1168391 A
- US 4328229 A
- KR 20020045417
- WO 2005105086 A

### Non-patent literature cited in the description

- *Cancer Research,* 1995, vol. 1, 839-845
- *Medical Research Review,* 2003, vol. 23 (4), 519-534
- *Cancer research,* 2001, 118-125